# EUROPEAN PATENT APPLICATION

(11) **EP 2 292 217 A2**
(43) Date of publication of application: **09.03.2011**
(21) Application number: 10175166.7
(22) Date of filing: 03.09.2010
(51) Int. Cl.: A61K 9/20, A61K 9/50, A61K 31/53

(54) **Modified release lamotrigine tablets**

(30) Priority: 03.09.2009 IN DE18172009
(71) Applicant: Ranbaxy Laboratories Limited, Gurgaon - 122001, Haryana (IN)
(72) Inventor: Bhavarisetti, Murali Krishna, 521185, Krishna, Andhra Paradesh (IN); Verma, Rajan K., 110043, New Delhi, Delhi (IN); Singh, Romi Barat, 221002, Varanasi, Uttar Pradesh (IN)
(74) Representative: Cronin, Brian Harold John

(57) **Abstract**

The present invention relates to uncoated modified-release tablets of lamotrigine and the process for preparation thereof, wherein the tablets comprise a portion of lamotrigine in an admixture with a pH-dependent polymer.

## Description

### Field of the Invention

The present invention relates to uncoated modified-release tablets of lamotrigine and process for preparation thereof, wherein the tablets include a portion of lamotrigine in an admixture with a pH-dependent polymer.

### Background of the Invention

Lamotrigine is an antiepileptic drug (AED) of the phenyltriazine class. Its chemical name is 3,5 -diamino-6-(2, 3-dichlorophenyl)-1,2,4-triazine, which is structurally designated as:

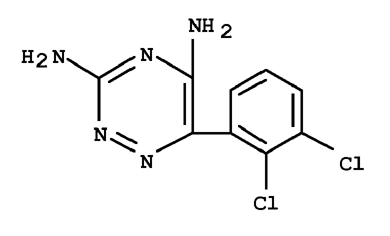

U.S. Patent No. 4,602,017 and European Patent No. EP 0 021 121 disclose lamotrigine or a pharmaceutically acceptable salt thereof, and also the methods using lamotrigine to treat convulsions and epilepsy. They also describes the process of preparation of lamotrigine. Lamotrigine is indicated for use as adjunctive therapy for partial seizures, the generalized seizures of Lennox-Gastaut syndrome, and primary generalized tonic-clonic seizures in adults and pediatric patients (≥2 years of age). It is also indicated for conversion to monotherapy in adults with partial seizures who are receiving treatment with carbamazepine, phenytoin, phenobarbital, primidone, or valproate as the single AED. Further, it is indicated for the maintenance treatment of Bipolar I Disorder to delay the time to occurrence of mood episodes (depression, mania, hypomania, mixed episodes) in patients treated for acute mood episodes with standard therapy.

Oral immediate-release and chewable dispersible tablet formulations of lamotrigine are commercially available in the United States under the brand names Lamictal® and Lamictal® CD from GlaxoSmithKline, USA. Lamotrigine is rapidly and completely absorbed after oral administration with negligible first-pass metabolism (absolute bioavailability is 98%). The bioavailability is not affected by food. Peak plasma concentrations occur anywhere from 1.4 to 4.8 hours following drug administration. It has been observed and well documented in the art that the existing immediate-release formulations of lamotrigine lead to severe side effects owing to a rapid increase in blood plasma levels after each dosing. In fact, serious rashes requiring hospitalization and discontinuation of treatment have been reported in association with the use of Lamictal®. To ameliorate these drawbacks associated with conventional immediate release formulations, lamotrigine is formulated into a controlled release once-daily dosage form which is available in the USA, as Lamictal® XR extended release tablets from GlaxoSmithKline Inc.

U.S. Application Publication No. 2004/0043996 discloses a multiparticulate controlled-release formulation, which comprises particles of lamotrigine, a release rate controlling polymer, and a rapidly disintegrating binder, which allows the particles to rapidly disperse in an aqueous environment. It relates to inert cores or particles, coated or layered, with drug and a rate controlling polymer to form a rate-controlling membrane or micromatrix. Additionally, it teaches that such coated particles can be further coated with an enteric polymer.

U.S. Application Publication No. 2004/0192690 describes a sustained-release tablet formulation comprising: (i) a matrix core containing lamotrigine and a release-retarding swellable and/or gellable polymer, and (ii) a semi-permeable barrier coating, thereby allowing diffusion control of drug-release by a water-insoluble polymer or a partially water-soluble polymer, wherein the outer barrier includes of one or more orifices extending from the outside of the coating substantially through the coating but not penetrating the core. Such orifices are generally drilled into the tablet by removing certain portions of the coating; typically, the orifices can be produced by mechanical drilling, ultrasonic cutting or laser. The orifices can be any shape, for example, oval, round, square or even shaped as text, for example, as a company logo. Such a formulation is referred as a Diffcore® device. Such devices are also described in U.S. Patent No. 5,004,614.

U.S. Application Publication No. 2009/0196924 discusses formulations of lamotrigine with a core and a coating. The formulation is described to be immediate-release or a matrix based extended-release core, which in turn is coated using rate-controlling pH-dependent and/or pH-independent polymer(s).

U.S. Application Publication No. 2009/0022789 discloses formulations, which comprise of lamotrigine admixed with a release-equalizing composition comprising a pharmaceutically acceptable organic acid and a release-enhancing polymer, which may be an enteric polymer. Such formulations further contain a release-retarding polymer and exhibit a significantly similar rate of release through the gastrointestinal tract.

In the above discussed prior art, once daily formulations of lamotrigine are coated dosage forms, wherein the coating is instrumental in achieving the desired release profile of lamotrigine from the dosage form. However, it is well known to a skilled artisan that a functional coating process in the manufacturing of a pharmaceutical dosage form is not only technically difficult but also requires expertise of skilled labor. It can be time consuming and expensive from the manufacturing point of view.

Therefore, there exists a need to formulate a simpler, time and cost efficient process for the manufacturing of once daily lamotrigine formulations. Accordingly, in the present case, the inventors have prepared a modified-release uncoated tablets of lamotrigine using techniques other than those discussed in the art, so as to formulate a dosage form which can be easily manufactured and that provides a gradual controlled-release of lamotrigine throughout the length of the gastrointestinal tract, *i.e*., with a portion of the drug releasing initially in the stomach and then continuing to release the remaining portion of the drug in the small intestine through the large intestine.

### Summary of the Invention

In one general aspect the present invention provides for a modified-release uncoated tablet of lamotrigine. The uncoated tablet includes:
(a) a first portion contains about 20% to about 90% of the lamotrigine in an admixture with a pH-dependent polymer; and
(b) a second portion includes the remaining amount of lamotrigine and a pH-independent polymer.

Embodiments of this aspect may include one or more of the following features. For example, the pH-independent polymer may be hydrophilic. Suitable pH-independent polymers include one or more of methylcellulose, ethylcellulose, hydroxymethyl cellulose, hydroxyethyl cellulose, hydroxypropyl cellulose, hydroxybutylcellulose, hydroxyethyl methylcellulose, hydroxypropyl methylcellulose, carboxymethylcellulose, sodium carboxymethylcellulose, carboxymethyl ethylcellulose, methacrylate copolymers, polyvinylalcohol; polyvinylpyrrolidone, copolymers of polyvinylpyrrolidone with vinyl acetate, combinations of polyvinylalcohol and polyvinylpyrrolidone; polyethylene oxide, polypropylene oxide, copolymers of ethylene oxide and propylene oxide.

The pH-dependent polymer may be present in an amount of not more than about 20% by weight of the tablet. Suitable pH-dependent polymers includes one or more of polyacrylic and polymethacrylic acids and polyacrylate and methacrylate based polymers; hydroxypropyl methylcellulose phthalate (HPMCP), cellulose acetate phthalate (CAP), ethylcellulose phthalate, cellulose acetate trimelliate (CAT), hydroxypropyl methylcellulose acetate succinate, cellulose acetate succinate, polyvinyl acetate phthalate (PVAP), polyvinyl acetaldiethylamino acetate, shellac.

The first and second portions are present in a ratio of 1:9 to 9:1. The tablet is a multi-layer tablet. The tablet may further include one or more of diluent(s), binder(s), lubricants(s), granulating solvent(s), glidants(s).

The tablet may have the following in vitro dissolution profile when measured in a USP type II apparatus, at 50 rpm, at a temperature of 37°C±0.5°C in 900mL of 0.1 N Hydrochloric acid medium:
(i) at most about 35% drug released in 2 hours;
(ii) at most about 55% drug released in 4 hours;
(iii) at most about 80% drug released in 8 hours; and
(iv) at least about 80% drug released in 16 hours.

### Detailed Description of the Invention

The term "modified-release tablets", as referred to herein, is defined to mean oral tablet dosage forms, which when administered, releases the active medicament at a relatively constant rate and provides a plasma concentration of the active medicament that remain substantially invariant with time within the therapeutic range of the active medicament over a 24-hour period. This definition includes "prolonged-release", "controlled-release", "extended-release", "delayed-release" and "sustained-release" tablet formulations. The formulations, as described herein, deliver a therapeutically effective amount of lamotrigine to a patient for at least up to 12 hours, particularly, up to 16 hours, more particularly, up to 20 hours following a once-daily administration. The once-daily administration of the tablets, as described herein, may exhibit the following *in vitro* dissolution profile when measured in a USP type II apparatus, at 50 rpm, at a temperature of 37°C±0.5°C in 900mL of 0.1 N hydrochloric acid medium:
(i) at most about 35% drug released in 2 hours;
(ii) at most about 55% drug released in 4 hours;
(iii) at most about 80% drug released in 8 hours; and
(iv) at least about 80% drug released in 16 hours.

The term "therapeutically effective amount" intends to describe an amount of the active agent which stops or reduces the progress of the condition to be treated or which otherwise completely or partly cures or acts palliatively on the condition. Lamotrigine or a pharmaceutically acceptable salt or derivative thereof may be present in an amount from about 1 mg to about 500 mg in the modified-release tablet. Particularly, the modified-release tablet may comprise of lamotrigine or a pharmaceutically acceptable salt or derivative thereof in an amount of 25mg, 50mg, 100mg, 150mg, 200mg, 250mg, and 300mg. The recommended dose of Lamictal® XR may also be considered as a standard dose.

The "modified-release tablets", as described herein, are uncoated. The term "uncoated" refers to the compressed tablets that are not processed with a coating composition which alters, changes, or influences the release characteristics of the active ingredient from the modified-release tablet. The tablets may be monolayer, as well as, multi-layer tablets. In one embodiment, the uncoated modified-release tablet is a bi-layer tablet.

The "first" and "second portions" of the modified-release tablet, as described herein, are distinct pharmaceutical compositions, which may include compressed tablets, mini tablets, granules, admixtures, homogeneous intimate or non-intimate blends, beads, powders, pellets, pills, and the like. Further, each of these portions may form a distinct layer or zone in the modified-release tablet, such that, one layer may enclose the other layer from all sides in a continuous manner, or each layer may be adjacent to each other.

The "first portion" of the modified-release tablet, as discussed herein, which includes from about 20% to about 90% of the lamotrigine in an admixture with a pH-dependent polymer.

The pH-dependent polymer in the first portion of the modified-release tablet may be in an admixture with at least about 20% but not more than about 90%, particularly, in an amount of about 40% but not more than about 80% of lamotrigine by weight of the total amount of lamotrigine. The pH-dependent polymer is present in an amount of not more than about 20% by weight of the modified-release tablet, particularly, not more than about 15% by weight of the modified-release tablet, more particularly not more than about 10% by weight of the modified-release tablet.

The admixture in the first portion of the modified-release tablet of the invention may further include a pH-independent polymer, which may be optionally in combination with one or more of other conventionally used pharmaceutically acceptable excipients. The amounts of the pH-dependent polymer and the pH-independent polymer present in the first portion of the modified-release tablet, as described herein, may be in a ratio of about 1:1 to about 1:40, particularly in a ratio about 1:1 to about 1:20, and more particularly in a ratio of about 1:1 to about 1:10.

The pH-dependent polymer may include polyacrylic and polymethacrylic acids and polyacrylate and methacrylate based polymers, and mixtures thereof, such as those available under the trade name Eudragit®, for example Eudragit® EPO, Eudragit® L 12.5, Eudragit® L 100, Eudragit® S 12.5, Eudragit® S 100, Eudragit® L100-55 or L30D-55 (methacrylic acid and ethyl acrylate copolymer) or the like (Evonik-Rohm Industries, Germany); reaction products such as hydroxypropyl methylcellulose phthalate (HPMCP), cellulose acetate phthalate (CAP), ethylcellulose phthalate, cellulose acetate trimellitate (CAT), hydroxypropyl methylcellulose acetate succinate, cellulose acetate succinate, and the like cellulose derivative such as an alkyl cellulose, a hydroxyalkyl cellulose, a hydroxyalkyl alkylcellulose or a cellulose ester with at least one polybasic acid such as succinic acid, maleic acid, ophthalic acid, tetrahydrophthalic acid, hexahydrophthalic acid, trimellitic acid or pyromellitic acid; polyvinyl acetate phthalate (PVAP), polyvinyl acetaldiethylamino acetate, shellac, or mixtures thereof. For example, the pH-dependent polymer is Eudragit® L100-55 or L30D-55.

The "second portion" of the modified-release tablet, as described herein, comprises of the remaining amount of lamotrigine and a pH-independent polymer. The pH-independent polymer may be a hydrophilic polymer with swellable and/or gellable properties in the aqueous media. Alternately, the pH-independent polymer may be hydrophobic.

The pH-independent polymer, may include alkylcelluloses, such as methylcellulose, ethylcellulose; hydroxyalkylcelluloses, for example, hydroxymethyl cellulose, hydroxyethyl cellulose, hydroxypropyl cellulose and hydroxybutylcellulose; hydroxyalkyl alkylcelluloses, such as hydroxyethyl methylcellulose and hydroxypropyl methylcellulose (hypromellose); carboxyalkylcelluloses, such as carboxymethylcellulose; alkali metal salts of carboxyalkylcelluloses, such as sodium carboxymethylcellulose; carboxyalkyl alkylcelluloses, such as carboxymethyl ethylcellulose; carboxyalkylcellulose esters; other natural, semi-synthetic, or synthetic polysaccharides, such as alginic acid, alkali metal and ammonium salts thereof, carrageenans, galactomannans, tragacanth, agar-agar, gum arabic, guar gum, xanthan gum, starches, pectins, such as sodium carboxymethyl amylopectin, chitin derivates, such as chitosan, polyfructans, inulin; polyacrylic acids and the salts thereof; polymethacrylic acids, and the salts thereof, methacrylate copolymers; polyvinylalcohol; polyvinylpyrrolidone (povidone), copolymers of polyvinylpyrrolidone with vinyl acetate; combinations of polyvinylalcohol and polyvinylpyrrolidone; polyalkylene oxides, such as polyethylene oxide and polypropylene oxide and copolymers of ethylene oxide and propylene oxide; and combinations thereof. The amount of pH-independent polymer in the second portion may vary from about 1 % to about 90% by weight of the modified-release tablet, in particular from about 10% to about 70% by weight of the modified-release tablet.

The second portion of the modified-release tablet may further include one or more of other conventionally used pharmaceutically acceptable excipients.

The term "pharmaceutically acceptable excipient(s)", as recited herein, includes conventional pharmaceutical additives known in the art, such as diluent(s), binder(s), lubricants(s), granulating solvent(s), glidants(s), or combinations thereof.

Suitable diluents include saccharides like lactose, dextrose, sucrose, fructose, maltose; sugars like mannitol, erythritol, sorbitol, xylitol and lactitol; cellulose derivatives like powdered cellulose, microcrystalline cellulose; dicalcium phosphate, tribasic calcium phosphate, calcium sulphate, calcium carbonate, kaolin, and the like.

Suitable binders include starch derivatives like corn starch and pregelatinized starch; cellulose ethers such as carboxymethyl cellulose, methylcellulose, hydroxypropyl cellulose, hydroxyethyl cellulose, hydroxypropyl methylcellulose; carboxy vinyl polymers like carbomers; acrylates such as Eudragits; polyvinylpyrrolidone (povidone), polyvinylpyrrolidone/vinyl acetate copolymer; xanthan gum, guar gum and other such materials routinely used in the art of solid dosage form manufacturing.

Suitable lubricants include magnesium stearate, calcium stearate, zinc stearate, sodium stearyl fumarate, powdered stearic acid, magnesium oleate, calcium palmitate, potassium laureate, sodium suberate, vegetable oil, mineral oil and the like. Glidants include talc, colloidal silicon dioxide, corn starch, and the like.

Suitable granulating solvents include water, ethanol, methanol, isopropyl alcohol, methylene chloride, acetone, and solutions of binder in these solvents.

The first and the second portions, as described herein, may be present in the modified-release tablet in a ratio of 1:9 to 9:1, particularly in a ratio of about 1:3 to about 3:1 by weight of the tablet.

The modified-release tablet, as described herein, may additionally include a third portion comprising additional active ingredients, for example, other antiepileptic drugs selected from phenytoin, phosphenytoin, trimethadione, carbamazepine, oxcarbazepine, valproates (valproic acid, sodium valproate, and divalproex sodium), tiagabine, levetiracetam, brivacetam, seletracetam, pregabalin, gabapentin, topiramate, beclamide, felbamate, zonisamide, valpromide, mesuximide, pheneturide and the like and/or other conventionally used pharmaceutically acceptable excipients, and combinations thereof.

The modified-release tablets may be formulated following any conventional techniques known in the art, namely dry granulation, aqueous or non-aqueous wet granulation, melt granulation, direct compression, roller compactation, pelletization, melting, intimate and non-intimate blending, kneading, and the like.

In one embodiment, a modified-release uncoated tablet of lamotrigine includes:
(a) granules comprising a portion of the total amount of lamotrigine in admixture with a pH-dependent polymer, diluent, binder, and pH-independent polymer; and
(b) a homogeneous blend comprising the remaining portion of the total amount of lamotrigine, pH-independent polymer, lubricant, and glidant.

In another embodiment, a modified-release uncoated tablet of lamotrigine comprises:
(a) granules comprising a portion of the total amount of lamotrigine in admixture with a pH-dependent polymer, diluent, binder, pH-independent polymer, lubricant, and glidant; and
(b) granules comprising the remaining portion of the total amount of lamotrigine, pH-independent polymer, diluent, binder, lubricant, and glidant.

In yet another embodiment, a modified-release uncoated tablet of lamotrigine is prepared by the following process:
(a) a portion of the total amount of lamotrigine is blended with a pH-dependent polymer, diluent and a pH-independent polymer;
(b) the blend of step (a) is granulated using a binder solution;
(c) the wet mass of step (b) is dried and sized;
(d) the granules obtained in step (c) are blended with a blend of the remaining portion of lamotrigine, pH-independent polymer, lubricant, and glidant; and
(e) the homogeneous blend of step (d) is compressed to tablets using appropriate tooling.

In another embodiment, a modified-release uncoated tablet of lamotrigine is prepared by the following process:
(a) a portion of the total amount of lamotrigine is blended with a pH-dependent polymer, diluent and pH-independent polymer;
(b) the blend of step (a) is granulated using a binder solution;
(c) the wet mass of step (b) is dried, sized and optionally, blended with lubricant and glidant;
(d) the remaining portion of lamotrigine is blended with pH-independent polymer, and diluent;
(e) the blend of step (d) is granulated using a binder solution;
(f) the wet mass of step (e) is dried, sized and optionally, blended with lubricant, glidant;
(g) the homogeneous blends of step (c) and (f) are optionally, blended with lubricant, glidant and the compressed to tablets using appropriate tooling.

In one aspect of the above embodiments, a suitable pH-dependent polymer is a methacrylic acid and ethyl acrylate copolymer.

In another aspect of the above embodiments, a suitable pH-independent polymer is one or more of hypromellose, hydroxyethyl cellulose, hydroxypropyl cellulose, acrylic acid polymers and copolymers.

In one aspect of the above embodiments, the tablet is a bi-layer tablet.

From the above disclosure it is apparent that various modifications and combinations of the formulations detailed in the text may be made without departing from the spirit and scope of the invention. The invention, as described herein, may be illustrated by the following examples, but should not to be construed to limit them.

### Example 1:

### Lot I

| **Stage** | **S.No.** | **Ingredients** | **mg/tab** |
|---|---|---|---|
| A | 1. | Lamotrigine | 60.00 |
| | 2. | Hypromellose K100LV | 30.00 |
| | 3. | Lactose | 30.00 |
| B | 1. | Povidone K 30 (PVP K30) | 6.00 |
| | 2. | Isopropyl Alcohol | qs |
| | **Total Weight** | | **126.00** |

### Lot II

| **Stage** | **S.No.** | **Ingredients** | **mg/tab** |
|---|---|---|---|
| C | 1. | Lamotrigine | 140.00 |
| | 2. | Hypromellose K100LV | 70.00 |
| | 3. | Lactose | 70.00 |
| | 4. | Eudragit® L100-55 | 15.00 |
| D | 1. | Povidone K 30 (PVP K30) | 14.00 |
| | 2. | Isopropyl Alcohol | qs |
| | **Total Weight** | | **309.00** |

### Composite Tablet:

| **Stage** | **S.No.** | **Ingredients** | **mg/tab** |
|---|---|---|---|
| E | 1. | Lot I | 126.00 |
| | 2. | Lot II | 309.00 |
| | 3. | Talc | 4.00 |
| | 4. | Magnesium Stearate | 4.00 |
| | **Total Weight of Tablet** | | **443.00** |

### Manufacturing Procedure:

### Lot I:

1. Ingredients A-1 to A-3 are passed through suitable sieve and transferred into a rapid mixer granulator and mixed at suitable impeller rpm for 5 minutes.
2. B-1 is dissolved in B-2 (isopropyl alcohol) under continuous stirring.
3. The premix of step 1 is granulated with the binder solution in step 2 using suitable process parameters.
4. The wet mass of step 3 is dried in fluid bed dryer and milled using a suitable screen and mill to obtain dried and sized granules.

### Lot II:

1. Ingredients C-1 to C-4 are passed through a suitable sieve and transferred into a rapid mixer granulator and mixed at suitable rpm for 5 minutes.
2. D-1 is dissolved in D-2 (isopropyl alcohol) under continuous stirring.
3. The premix of step 1 is granulated with binder solution of step 2 using suitable process parameters.
4. The wet mass of step 3 is dried in fluid bed dryer and milled using a suitable screen and mill to obtain dried and sized granules.

### Composite Tablet:

1. The granules of step-4 of Lot-I and step-4 of Lot-II are transferred into a suitable blender and blended together for a suitable duration.
2. Lubricants E-3 and E-4 are passed through a suitable sieve and are blended with the composite blend of step 1. The lubricated blend is compressed using 10.5 mm circular shaped punches.

### Example 2:

### Lot I

| **Stage** | **S.No.** | **Ingredients** | **mg/tab** |
|---|---|---|---|
| A | 1. | Lamotrigine | 70.00 |
| | 2. | Hypromellose K100LV | 35.00 |
| | 3. | Lactose | 30.00 |
| B | 1. | Purified water | qs |
| | **Total Weight** | | **135.00** |

### Lot II

| **Stage** | **S.No.** | **Ingredients** | **mg/tab** |
|---|---|---|---|
| C | 1. | Lamotrigine | 130.00 |
| | 2. | Hypromellose K100LV | 60.00 |
| | 3. | Lactose | 70.00 |
| D | 1. | Eudragit® L 30D-55* | 20.80 |
| | 2. | Purified Water | qs |
| | **Total Weight** | | **280.80** |

| | | | |
|---|---|---|---|
| Note: *mg/tab of Eudragit® L30D-55 is given as solids; dispersion weight is equivalent to 69.33 mg/tablet. | | | |

### Composite Tablet:

| **Stage** | **S.No.** | **Ingredients** | **mg/tab** |
|---|---|---|---|
| E | 1. | Lot I | 135.00 |
| | 2. | Lot II | 280.80 |
| | 3. | Talc | 4.00 |
| | 4. | Magnesium Stearate | 4.00 |
| | **Total Weight of Tablet** | | **423.80** |

### Manufacturing Procedure:

### Lot I:

1. Ingredients A-1 to A-3 are passed through suitable sieve and transferred into a rapid mixer granulator and mixed at a suitable impeller rpm for 5 minutes.
2. The premix of step 1 is granulated with B-1(purified water) using suitable process parameters.
3. The wet mass in step 2 is dried in fluid bed dryer and milled using a suitable screen and mill.

### Lot II:

1. Ingredients C-1 to C-3 are passed through a suitable sieve and transferred into fluid bed process (Top Spray).
2. D-1 is diluted with purified water (D-2).
3. The blend of step 1 is granulated using the granulating solution of step 2 in a fluid bed process using a top spray gun, followed by drying and sieving using a suitable sieve to de-aggregate lumps or agglomerates.

### Composite Tablet:

1. The granules of step-3 of Lot-I and step-3 of Lot-II are transferred into a suitable blender and blended together for suitable duration.
2. Lubricants E-3 and E-4 are passed through a suitable sieve and blended with step 1 of the composite blend. This lubricated blend is compressed using 10.5 mm circular shaped punches.

### Example 3:

| **Stage** | **S.No.** | **Ingredients** | **mg/tab** |
|---|---|---|---|
| A | 1. | Lamotrigine | 160.00 |
| | 2. | Hypromellose K100LV | 60.00 |
| | 3. | Hypromellose E4M | 20.00 |
| | 4. | Eudragit® L100-55 | 15.00 |
| | 5. | Microcrystalline Cellulose (Avicel® PH 101) | 80.00 |
| B | 1. | Povidone K30 (PVP K30) | 15.00 |
| | 2. | Isopropyl Alcohol | qs |
| C | 1. | Lamotrigine | 40.00 |
| | 2. | HPMC K100LV | 30.00 |
| D | 1. | Talc | 4.00 |
| | 2. | Magnesium Stearate | 4.00 |
| | **Total Tablet Weight** | | **428.00** |

### Manufacturing Procedure:

1. Ingredients A-1 to A-5 are passed through a suitable sieve and transferred into a rapid mixer granulator, and mixed at a suitable impeller rpm for 5 minutes.
2. B-1 is dissolved in B-2 (isopropyl alcohol) under continuous stirring.
3. The premix of step 1 is granulated with binder solution of step 2 using suitable process parameters.
4. The wet mass of step 3 is dried in fluid bed drier and milled using a suitable screen and mill.
5. Ingredients C-1 and C-2 are passed through a suitable sieve and transferred to a suitable blender and blended with the milled granules of step 4.
6. Lubricants D-1 and D-2 are passed through a suitable sieve and blended with the blend of step 5. This lubricated blend is compressed using 10.5 mm circular shaped punches.

### Example 4:

### Layer I

| **Stage** | **S.No.** | **Ingredients** | **mg/tab** |
|---|---|---|---|
| A | 1. | Lamotrigine | 50.00 |
| | 2. | Hypromellose K100LV | 25.00 |
| | 3. | Lactose | 25.00 |
| B | 1. | Povidone K 30 | 5.00 |
| | 2. | Isopropyl alcohol | qs |
| C | 1. | Talc | 1.00 |
| | 2. | Magnesium Stearate | 1.00 |
| | **Total Weight** | | **107.00** |

### Layer II

| **Stage** | **S.No.** | **Ingredients** | **mg/tab** |
|---|---|---|---|
| D | 1. | Lamotrigine | 150.00 |
| | 2. | Hypromellose K100LV | 75.00 |
| | 3. | Lactose | 75.00 |
| | 4. | Eudragit® L100-55 | 15.00 |
| E | 1. | Povidone K 30 | 15.00 |
| | 2. | Isopropyl alcohol | qs |
| F | 1. | Talc | 3.00 |
| | 2. | Magnesium stearate | 3.00 |
| | **Total Weight** | | **336.00** |

### Manufacturing Procedure:

### Layer I:

1. Ingredients A-1 to A-3 are passed through a suitable sieve and transferred into a rapid mixer granulator and mixed at a suitable impeller rpm for 5 minutes.
2. B-1 is dissolved in B-2 (Isopropyl alcohol) under continuous stirring.
3. The premix of step 1 is granulated with the binder solution of step 2 using suitable process parameters.
4. The wet mass of step 3 is dried in fluid bed dryer and milled using a suitable screen and mill.
5. Lubricants C-1 and C-2 are passed through suitable sieve and blended with the milled granules of step 4 in a blender for a suitable duration.

### Layer II:

1. Ingredients D-1 to D-4 are passed through suitable sieve and transferred into rapid mixer granulator and mixed at a suitable rpm for 5 minutes.
2. E-1 is dissolved in E-2 (isopropyl alcohol) under continuous stirring.
3. The premix of step 1 is granulated with binder solution of step 2 using suitable process parameters.
4. The wet mass of step-3 is dried in fluid bed dryer and milled suing a suitable screen and mill.
5. Lubricants F-1 and F-2 are passed through a suitable sieve and blended with milled granules of step-4 in a blender for a suitable duration.

### Compression Tooling:

Layer I and Layer II are compressed in a bi-layer tablet machine using 10.5 mm circular shaped punches.

### Examples 5 and 6:

### Lot I

| **S.No.** | **Ingredients** | **Quantity (mg/tab)** | |
|---|---|---|---|
| | | **Example 5** | **Example 6** |
| 1. | Lamotrigine | 80.00 | 120.00 |
| 2. | Hypromellose K100LV Premium | 40.00 | 60.00 |
| 3. | Microcrystalline Cellulose | 40.00 | 60.00 |
| 4. | Povidone (PVP K30) | 8.40 | 12.60 |
| 5. | Isopropyl Alcohol | qs | qs |
| **Total Weight** | | **168.40** | **252.60** |

### Lot II

| **S.No.** | **Ingredients** | **Quantity (mg/tab)** | |
|---|---|---|---|
| | | **Example 5** | **Example 6** |
| 1. | Lamotrigine | 120.00 | 80.00 |
| 2. | Hypromellose K100LV Premium | 60.00 | 40.00 |
| 3. | Eudragit® L100-55 | 9.00 | 6.00 |
| 4. | Microcrystalline Cellulose | 60.00 | 40.00 |
| 5. | Povidone (PVP K30) | 12.60 | 8.40 |
| 6. | Isopropyl alcohol | qs | qs |
| **Total Weight** | | **261.60** | **174.40** |

### Composite Tablet:

| **S.No.** | **Ingredients** | **Quantity (mg/tab)** | |
|---|---|---|---|
| | | **Example 5** | **Example 6** |
| 1. | Lot I | 168.40 | 252.60 |
| 2. | Lot II | 261.60 | 174.40 |
| 3. | Talc | 4.00 | 4.00 |
| 4. | Magnesium Stearate | 4.00 | 4.00 |
| **Total Weight of the Tablet** | | **438.00** | **435.00** |

### General Manufacturing Procedure for Examples 5 and 6:

### Preparation of Lot I:

Lamotrigine, Hypromellose K100LV Premium, and microcrystalline cellulose were passed through BSS#30 and transferred in to a Rapid Mixer Granulator, mixed at a suitable impeller speed for a suitable duration. The premix formed was granulated with a solution of povidone in isopropyl alcohol using suitable process parameters. The wet granular mass formed was dried in a fluid bed dryer at a suitable temperature until reaching the desired LOD. The granules formed were sized.

### Preparation of Lot II:

Lamotrigine, Hypromellose K100LV Premium, Eudragit® L100-55 and microcrystalline cellulose were passed through BSS#30 and transferred into a Rapid Mixer Granulator, mixed at a suitable impeller speed for suitable duration. The premix formed was granulated with a solution of povidone in isopropyl alcohol using a suitable process parameters. The wet granular mass formed was dried in a fluid bed dryer at suitable temperature until reaching the desired LOD. The granules formed were sized.

### Preparation of Composite Tablet:

The granules of Lot I and Lot II were blended for a suitable duration in a blender. The lubricants, talc and magnesium stearate, were added to this blend and then further blended for a suitable duration. The homogeneous blend obtained was compressed as tablets using appropriate punch tools.

The tablets of Example 5 and Example 6 were subjected to *in-vitro* dissolution studies in a USP type II apparatus, at 50 rpm, at a temperature of 37°C±0.5°C in 900mL of 0.1 N hydrochloric acid medium. Aliquot samples were withdrawn at predetermined time intervals and replaced with an equal amount of fresh media. The samples were processed and analyzed. The dissolution profiles of these tablets are provided in Table 1.

**Table 1: In-vitro release pattern of lamotrigine from controlled-release tablets prepared as per composition of Example 5 and Example 6 in USP II apparatus in 900mL of 0.1 N hydrochloric acid medium at 50 rpm at a temperature of 37°C±0.5°C.**

| **Time (h)** | **Percent of lamotrigine released from the composition prepared as per** | |
|---|---|---|
| | **Example 5** | **Example 6** |
| 1 | 18 | 18 |
| 2 | 27 | 27 |
| 4 | 44 | 42 |
| 6 | 60 | 56 |
| 8 | 73 | 68 |
| 10 | 90 | 90 |
| 12 | 96 | 94 |
| 16 | 95 | 98 |
| 20 | 95 | 98 |
| 24 | 97 | 97 |

## Claims

1. A modified-release uncoated tablet of lamotrigine comprising:
(a) a first portion consisting essentially of about 20% to about 90% of the lamotrigine in an admixture with a pH-dependent polymer; and
(b) a second portion comprising the remaining amount of lamotrigine and a pH-independent polymer.

2. The modified-release uncoated tablet according to claim 1, wherein the pH-independent polymer comprises hydrophilic.

3. The modified-release uncoated tablet according to claim 1, wherein the pH-independent polymer comprises one or more of methylcellulose, ethylcellulose, hydroxymethyl cellulose, hydroxyethyl cellulose, hydroxypropyl cellulose, hydroxybutylcellulose, hydroxyethyl methylcellulose, hydroxypropyl methylcellulose, carboxymethylcellulose, sodium carboxymethylcellulose, carboxymethyl ethylcellulose, methacrylate copolymers, polyvinylalcohol; polyvinylpyrrolidone, copolymers of polyvinylpyrrolidone with vinyl acetate, combinations of polyvinylalcohol and polyvinylpyrrolidone; polyethylene oxide, polypropylene oxide, copolymers of ethylene oxide and propylene oxide.

4. The modified-release uncoated tablet according to claim 1, wherein the pH-dependent polymer is present in an amount of not more than about 20% by weight of the tablet.

5. The modified-release uncoated tablet according to claim 1, wherein the pH-dependent polymer comprises one or more of polyacrylic and polymethacrylic acids and polyacrylate and methacrylate based polymers; hydroxypropyl methylcellulose phthalate (HPMCP), cellulose acetate phthalate (CAP), ethylcellulose phthalate, cellulose acetate trimelliate (CAT), hydroxypropyl methylcellulose acetate succinate, cellulose acetate succinate, polyvinyl acetate phthalate (PVAP), polyvinyl acetaldiethylamino acetate, shellac.

6. The modified-release uncoated tablet according to claim 1, wherein the first and second portions are present in a ratio of 1:9 to 9:1.

7. The modified-release uncoated tablet according to claim 1, wherein the tablet is a multi-layer tablet.

8. The modified-release uncoated tablet according to claim 1, wherein the tablet further comprises one or more of diluent(s), binder(s), lubricants(s), granulating solvent(s), glidants(s).

9. The modified-release uncoated tablet according to claim 1, wherein the tablet comprises the following *in vitro* dissolution profile when measured in a USP type II apparatus, at 50 rpm, at a temperature of 37°C±0.5°C in 900mL of 0.1 N Hydrochloric acid medium:
(i) at most about 35% drug released in 2 hours;
(ii) at most about 55% drug released in 4 hours;
(iii) at most about 80% drug released in 8 hours; and
(iv) at least about 80% drug released in 16 hours.
